# EUROPÄISCHE PATENTANMELDUNG

(11) **EP 1 922 931 A2**
(43) Veröffentlichungstag der Anmeldung: **21.05.2008**
(21) Anmeldenummer: 07120657.7
(22) Anmeldetag: 17.01.2002
(51) Int. Cl.: A01N 63/02, A01P 7/00, A01N 61/00

(54) **Verfahren zur Kontrolle von Schadorganismen in Nutzpflanzenkulturen**

(30) Priorität: 03.02.2001 DE 10104871
(62) Teilanmeldung aus: 02708294.0
(71) Anmelder: BASF SE, 67056 Ludwigshafen (DE)
(72) Erfinder: Kern, Manfred, 55296 Lörzweiler (DE)

(57) **Zusammenfassung**

Verfahren zur Bekämpfung von Schadorganismen in gentechnisch veränderten Gemüsepflanzen, die ein aus Bacillus thuringiensis abgeleitetes Gen enthalten, welches für ein insektizid wirksames Protein codiert und dieses exprimiert, dadurch gekennzeichnet, dass man eine insektizid wirksame Menge an einer oder mehreren Verbindungen aus den folgenden Gruppen (a) bis (f) auf die Pflanzen, deren Saat- oder Vermehrungsgut und/oder deren Anbaufläche aufbringt:
a) insektizide Organophosphorverbindungen;
b) Pyrethroide;
c) insektizide Carbamate;
d) Biopestizide;
e) insektizide Wachstumsregulatoren;
f) Sonstige.

Das erfindungsgemäße Verfahren ermöglicht eine reduzierte Aufwandmenge an Pflanzenschutzmitteln, die synergistisch mit den transgenen Pflanzen wirken, sowie eine Erhöhung und Verbreiterung des Wirkungsgrades der transgenen Pflanzen und bietet somit ökonomische wie ökologische Vorteile.

## Beschreibung

Die Erfindung betrifft ein Verfahren zur Bekämpfung von Schadorganismen in Kulturen von Bt-Gemüse.

Genetisch veränderte Gemüsepflanzen wie Auberginen, die Toxine aus Bacillus thuringiensis (Bt) exprimieren und dadurch eine Resistenz gegen Befall durch bestimmte Schadinsekten aufweisen (Bt-Gemüse), sind bekannt und werden bereits im kommerziellen Pflanzenbau eingesetzt (siehe z. B. US-A 6,072,105). Obwohl solch gentechnisch verändertes Gemüse bereits sehr gute Eigenschaften aufweist, bestehen doch noch eine Reihe von Problemen, so dass ein breiter Raum für Verbesserungen bleibt.

Aufgabe war es daher weiterhin, möglichst wirksame und umweltverträgliche Problemlösungen zur Bekämpfung von Gemüseschädlingen bereitzustellen.

Es wurde nun überraschend gefunden, dass bestimmte Klassen von Insektiziden bei Verwendung in Kombination mit Bt-Gemüse synergistische Effekte aufweisen.

Gegenstand der Erfindung ist daher ein Verfahren zur Bekämpfung von Schadorganismen in gentechnisch veränderten Gemüsepflanzen, die ein aus Bacillus thuringiensis abgeleitetes Gen enthalten, welches für ein insektizid wirksames Protein codiert und dieses exprimiert, wobei man eine insektizid wirksame Menge an einer oder mehreren Verbindungen aus den folgenden Gruppen (a) bis (f) auf die Pflanzen, deren Saat- oder Vermehrungsgut und/oder deren Anbaufläche aufbringt:
a) insektizide Organophosphorverbindungen aus der Gruppe:
   Acephate (4), Azinphos-ethyl (40), Azinphos-methyl (41), Cadusafos (101), Chlorfenvinphos (124), Chlormephos (128), Chlorpyrifos (137), Demeton-S-methyl (205), Diazinon (209), Dicrotophos (223), Dimethoate (243), Disulfoton (257), Ethion (283), Ethoprophos (286), Etrimfos (295), Fonofos (366), Isazofos (429), Isofenphos (430), Malathion (448), Methamidophos (479), Methidathion (481), Mevinphos (499), Monocrotophos (502), Omethoate (533), Parathion (551), Phenthoate (565), Phorate (570), Phosalone (571), Phosmet (572), Phosphamidon (573), Phoxim (575), Pirimiphosmethyl (585), Profenofos (594), Prothiofos (614), Pyridaphenthion (625), Quinalphos (634), Terbufos (690), Tetrachlorvinphos (694) und Triazophos (726);
b) Pyrethroide aus der Gruppe:
   Acrinathrin (9), Allethrin (17), Bifenthrin (70), Cycloprothrin (172), Cyfluthrin (176), (Beta)-Cyfluthrin (177), (Lambda)-Cyhalothrin (180), Cypermethrin (183), (Alpha)-Cypermethrin (184), (Beta)-Cypermethrin (185), (Zeta)-Cypermethrin (187), Deltamethrin (204), Esfenvalerate (277), Fenpropathrin (312), Fenvalerate (319), Flucythrinate (333), (Tau)-Fluvalinate (362), Permethrin (561), Tefluthrin (687), Tralomethrin (718) und ZXI 8901 (759);
c) insektizide Carbamate aus der Gruppe:
   Alanycarb (15), Aldicarb (16), Amitraz (22), Bendiocarb (56), Benfuracarb (58), Butocarboxim (95), Carbaryl (106), Carbofuran (109), Carbosulfan (110), Ethiofencarb (282), Formetanate (369), Furathiocarb (376), Isoprocarb (431), Methiocarb (482), Methomyl (483), Oxamyl (541), Pirimicarb (583), Propoxur (610), Thiofanox (709), Thiodicarb (708) und Trimethacarb (743);
d) Biopestizide aus der Gruppe:
   Bacillus thuringiensis (46, 47), Bacillus firmus, Granulose und Kernpolyeder-Viren, Beauveria bassiana (52), Beauveria brogniartii (53) und Baculoviren, wie Autographa california;
e) insektizide Wachstumsregulatoren aus der Gruppe:
   Chlorfluazuron (125), DBI-3204, Diflubenzuron (231), Flucycloxuron (332), Flufenoxuron (335), Hexaflumuron (399), Lufenuron (446), Novaluron (527), Methoxyfenozide (643), Teflubenzuron (686), Tebufenozide (679) und Triflumuron (739);
f) Sonstige:
   Abamectin (1), Bensultap (64), Cartap (113), Chlordane (121), Chlorfenapyr (123), DNOC (261), Endosulfan (270), Fipronil (323), Ethiprole, Imidacloprid (418), Thiacloprid, Phosphine (574), Oleinsäure/Fettsäuren (532), Pymetrozine (615), Thiocyclam (707), IKI-220, Tolfenpyrad (NEW), Acetamiprid (5), Spinosad (754), Silafluofen (650) und Thiamethoxam (NEW).

Die eingeklammerten Zahlen geben die Eintragungsnummer in 'The e-Pesticide Manual', CD-ROM-Version 1.1,1999-2000 (ISBN: 1-901396-22-3), basierend auf 'The Pesticide Manual', 11 th Ed., British Crop Protection Council, Farnham 1997, wieder.

Baculoviren sind beispielsweise beschrieben in J. Ind. Microbiol. & Biotech (1997) 19:192. Bacillus firmus, DBI-3204, Thiacloprid und IKI-220 sind beispielsweise beschrieben in Proceedings of the BCPC-Conference, Pest & Diseases, 2000.
Auf diese Quellen sowie die darin zitierte Literatur wird hiermit ausdrücklich Bezug genommen, sie gelten durch Zitat als Bestandteil der Beschreibung.

Das erfindungsgemäße Verfahren ermöglicht eine reduzierte Aufwandmenge an Pflanzenschutzmitteln, die synergistisch mit den transgenen Pflanzen wirken, sowie eine Erhöhung und Verbreiterung des Wirkungsgrades der transgenen Pflanzen und bietet somit ökonomische wie ökologische Vorteile.

Die Vorteile des erfindungsgemäßen Verfahrens liegen einerseits in Synergismen mit den in der transgenen Pflanze produzierten Bacillus thuringiensis Toxinen (Bt-Toxinen) sowie andererseits in einer z. B. verminderten Anzahl von Applikationen oder einer Reduzierung der Aufwandmengen auf zum Teil subletable Dosierungen (im Vergleich zur konventionellen Anwendung der einzelnen Insektizide) und einer damit verbundenen deutlich reduzierten Umweltbelastung.

Insbesondere Kombinationen der genannten Wirkstoffe zeigen zusammen mit den endogenen, d. h. innerhalb der transgenen Pflanzen, produzierten Bt-Toxinen eine ausgeprägte synergistische Wirkung auf eine Vielzahl von zu bekämpfenden Schadorganismen.

Ebenso Gegenstand der Erfindung ist die Verwendung von Verbindungen aus den oben genannten Gruppen (a) bis (f) zur Bekämpfung von Schadorganismen in gentechnisch veränderten Gemüsepflanzen, die ein aus Bacillus thuringiensis abgeleitetes Gen enthalten, welches für ein insektizid wirksames Protein codiert und dieses exprimiert.

Der Begriff "insektizid wirksam" umfasst im Sinne der Erfindung insektizide, akarizide, molluskizide, nematizide, ovizide Wirkung, sowie eine abschreckende (repellent), verhaltensändernde (behavior modifier) und sterilisierende (sterilant) Wirkung.

Bevorzugte insektizide Wirkstoffe sind die genannten insektiziden Organophosphorverbindungen, Pyrethroide, insektiziden Carbamate, insektiziden Wachstumsregulatoren, Abamectin, Acetamiprid, Chlorfenapyr, Endosulfan, Fipronil, Ethiprole, Imidacloprid, Pymetrozine, Spinosad, Silafluofen, Thiamethoxam, Thiacloprid, Tolfenpyrad, IKI-220 und Bacillus thuringiensis.

Besonders bevorzugt sind Ethoprophos, Malathion, Parathion, Phosalone, Pirimiphosmethyl, Triazophos, Acrinathrin, Deltamethrin, Tefluthrin, Aldicarb, Amitraz, Bendiocarb, Carbaryl, Carbofuran, Oxamyl, Pirimicarb, Thiodicarb, Diflubenzuron, Lufenuron, Novaluron, Methoxyfenozide, Tebufenozide, Abamectin, Acetamiprid, Chlorfenapyr, Endosulfan, Fipronil, Ethiprole, Imidacloprid, Pymetrozine, Spinosad, Silafluofen, Thiamethoxam, Thiacloprid, Tholfenpyrad, IKI-220 und Bacillus thuringiensis.
Ganz besonders bevorzugt sind Thiodicarb, Acetamiprid, Fipronil und Tolfenpyrad.

Bevorzugt sind auch Mischungen aus zwei oder mehr, vorzugsweise zwei oder drei, besonders bevorzugt zwei, der insektizid wirksamen Verbindungen.

Besonders bevorzugt sind Mischungen der genannten Organophosphorverbindungen mit den genannten Pyrethroiden.

Ganz besonders bevorzugt sind die im folgenden aufgeführten Mischungen:
Deltamethrin und Endosulfan, Deltamethrin und Spinosad, Deltamethrin und Chlorphenapyr, Deltamethrin und Bacillus thuringiensis, Deltamethrin und Methoxyfenozide, Deltamethrin und Tebufenozide, Endosulfan and Amitraz, Endosulfan und Bacillus thuringiensis, Cyfluthrin und Chlorpyriphos.

Ebenso bevorzugt sind Mischungen der genannten Pyrethroide mit Imidacloprid, sowie Mischungen der genannten Pyrethroide mit Tebufenozide.

Die erfindungsgemäß eingesetzten insektizid wirksamen Verbindungen sind bekannt und überwiegend kommerziell erhältlich.

Die erfindungsgemäß verwendeten Insektizide sind üblicherweise als kommerzielle Formulierungen erhältlich. Sie können aber gegebenenfalls auf verschiedene Art formuliert werden, je nachdem wie es durch die biologischen und/oder chemischphysikalischen Parameter vorgegeben ist. Als Formulierungsmöglichkeiten kommen beispielsweise in Frage:
Spritzpulver (WP), emulgierbare Konzentrate (EC), wässrige Lösungen (SL), Emulsionen, versprühbare Lösungen, Dispersionen auf Öl- oder Wasserbasis (SC), Suspoemulsionen (SE), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und beispielsweise beschrieben in:
Winnacker-Küchler, 1986, "Chemische Technologie", Band 7, 4. Aufl., C. Hauser Verlag München; van Falkenberg, 1972-73, "Pesticides Formulations", 2nd Ed., Marcel Dekker N.Y.; Martens, 1979, "Spray Drying Handbook", 3rd Ed., G. Goodwin Ltd. London.

Die notwendigen Formulierungshilfsmittel, wie Inertmaterialien, Tenside, Lösungsmittel und weitere Zusatzstoffe, sind ebenfalls bekannt und werden beispielsweise beschrieben in:
Watkins, "Handbook of Insecticide Dust Diluents and Carriers", 2nd Ed., Darland Books, Caldwell N.J.; v. Olphen, "Introduction to Clay Colloid Chemistry", 2nd Ed., J. Wiley & Sons, N.Y.; Marsden, "Solvents Guide", 2nd Ed., Interscience, N.Y. 1950; Mc-Cutcheon's, "Detergents and Emulsifiers Annual", MC Publ. Corp., Ridgewood, N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1967; Winnacker-Küchler, "Chemische Technologie", Band 7, 4. Aufl., C. Hanser Verlag München 1986.

Auf der Basis dieser Formulierungen lassen sich auch Kombinationen mit anderen pestizid wirksamen Stoffen, Düngemitteln und/oder Pflanzenwachstumsregulatoren herstellen, z. B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z. B. polyoxethylierte Alkylphenole, polyoxethylierte Fettalkohole, Alkyl- oder Alkylphenol-sulfonate und Dispergiermittel, z. B. ligninsulfonsaures Natrium, 2,2'-dinaphthylmethan-6,6'-disulfonsaures Natrium enthalten.

Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Ca-Dodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z. B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z. B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstoffformulierungen gegebenenfalls die jeweils üblichen haft-, netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. w. variiert die erforderliche Aufwandmenge. Sie kann innerhalb weiter Grenzen schwanken, z. B. zwischen 0,1 g/ha und 5,0 kg/ha oder mehr Aktivsubstanz. Vorzugsweise liegt sie jedoch zwischen 0,1 g/ha und 1,0 kg/ha. Auf Grund der synergistischen Effekte zwischen Bt-Gemüse und Insektizid sind Aufwandmengen von 0,1 bis 500 g/ha, besonders bevorzugt.

Für insektizide Organophosphorverbindugnen (a) sind Aufwandmengen von 50 bis 500 g/ha bevorzugt, besonders bevorzugt sind 50 bis 200 g/ha.

Für Pyrethroide (b) sind Aufwandmengen von 0,1 bis 10 g/ha bevorzugt, besonders bevorzugt sind 0,1 bis 6,0 g/ha.

Für insektizide Carbamate (c) sind Aufwandmengen von 50 bis 5000 g/ha bevorzugt, besonders bevorzugt sind 50 bis 2000 g/ha.

Für Biopestizide (d) sind die handelsüblichen Aufwandmengen bevorzugt.

Für insektizide Wachstumsregulatoren (e) sind Aufwandmengen von 10 bis 1000 g/ha bevorzugt, besonders bevorzugt sind 50 bis 500 g/ha.

Für die Insektizide der Gruppe (f) sind Aufwandmengen von 0,1 bis 5000 g/ha bevorzugt, besonders bevorzugt sind 0,1 bis 300 g/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Bevorzugte weitere Mischungspartner sind
1. aus der Gruppe der Carbonsäureester Alphametrin, 5-Benzyl-3-furylmethyl-(E)-, (1 R)-cis-2,2-di-methyl-3-(2-oxothiolan-3-ylidenemethyl)cyclopropanecarboxylate, Bioallethrin, Bioallethrin((S)-cyclopentylisomer), Bioresmethrin, (RS)-1-Cyano-1-(6-phenoxy-2-pyridyl)methyl-(1 RS)-trans-3-(4-tert.butylphenyl)-2,2-dimethylcyclopropanecarboxylate (NCI 85193), Cythithrin, Cyphenothrin, Empenthrin, Fenfluthrin, Flumethrin, Fluvalinate (D-Isomer), Imiprothrin (S-41311), Phenothrin ((R)-Isomer), Prallethrin, Pyrethrine (natürliche Produkte), Resmethrin, Tetramethrin, Theta-Cypermethrin (TD-2344), Transfluthrin;
2. aus der Gruppe der Amide Chlordimeform;
3. Sonstige:
   ABG-9008, Anagrapha falcitera, AKD-1 022, AKD-3059, Bifenazate (D-2341), Binapacryl, BJL-932, Bromopropylate, BTG-504, BTG-505, Buprofezin, Camphechlor, Chlorobenzilate, 2-(4-Chlorphenyl)-4,5-diphenylthiophen (UBI-T 930), Chlorfentezine, Chromafenozide (ANS-118), CG-216, CG-217, CG-234, A-184699, Cyclopropancarbonsäure-(2-naphthylmethyl)ester (Ro12-0470), Cyromazin, Diacloden, N-(3,5-Dichlor-4-(1,1,2,3,3,3-hexafluor-1-propyloxy)phenyl)carbamoyl)-2-chlorbenzcarboximidsäureethylester, Dicofol, N-(2,3-Dihydro-3-methyl-1,3-thiazol-2-ylidene)-2,4-xylidine, Dinobuton, Dinocap, Diofenolan, DPX-062, Emamectin-Benzoate (MK-244), Sulfethiprole, Ethofenprox, Etoxazole (YI-5301), Fenoxycarb, Fluazuron, Flumite (Flufenzine, SZI-121), 2-Fluoro-5-(4-(4-ethoxyphenyl)-4-methyl-1-pentyl)diphenylether (MTI 800), Fenpyroximate, Fenthiocarb, Flubenzimine, Flufenprox (ICI-A5683), Fluproxyfen, Gamma-HCH, Halofenozide (RH-0345), Halofenprox (MIT-732), Hexythiazox, HOI-9004, Hydramethylnon (AC 217300), Indoxacarb (DPX-MP062), Kanemite (AKD-2023), M-020, MIT-446, Ivermectin, M-020, Milbemectin, NC-196, Neemgard, Nitenpyram (TI-304), 2-Nitromethyl-4,5-dihydro-6H-thiazin (DS 52618), 2-Nitromethyl-3,4-dihydrothiazol (SD 35651), 2-Nitromethylene-1,2-thiazinan-3-ylcarbamaldehyde (WL 108477), Pyriproxyfen (S-71639), NC-196, NC-1111, NNI-9768, OK-9701, OK-9601, OK-9602, Propargite, Pyridaben, Pyrimidifen (SU-8801), RH-0345, RYI-210, S-1283, S-1833, SB7242, SI-8601, Silomadine (CG-177), SU-9118, Tebufenpyrad (MK-239), Tetradifon, Tetrasul, Tl-435, Verbutin, Vertalec (Mykotal), YI-5301.

Der Wirkstoffgehalt der aus den handelsüblichen Formulierungen bereiteten Anwendungsformen kann von 0,00000001 bis zu 95 Gew.-%, vorzugsweise zwischen 0,00001 und 1 Gew.-%, Wirkstoff liegen.

Beispielsweise enthalten entsprechende Formulierungen von Mischungen aus Pyrethroiden und Organophosphorverbindungen 0,05 bis 0,01 Gew.-% Pyrethroid und 0,25 bis 0,20 Gew.-% Organophosphorverbindung, bevorzugt 0,01 bis 0,001 Gew.-% Pyrethroid und 0,2 bis 0,1 Gew.-% Organophosphorverbindung.

Für Mischungen aus Pyrethroiden und Endosulfan ist ein Verhältnis von 0,05 bis 0,01 Gew.-% Pyrethroid zu 0,7 bis 0,2 Gew.-% Endosulfan bevorzugt, besonders bevorzugt sind 0,01 bis 0,001 Gew.-% Pyrethroid und 0,35 bis 0,2 Gew.-% Endosulfan.

Für Mischungen aus Pyrethroiden und Bacillus thuringiensis (Bt) gelten die oben für Pyrethroide angegebenen Werte, der Bt-Anteil beträgt vorzugsweise 0,01 bis 0,001 Gew.-%, besonders bevorzugt 0,005 bis 0,001 Gew.-%.

Mischungen aus Endosulfan und Amitraz enthalten vorzugsweise 0,35 bis 0,2 Gew.-% Endosulfan und 0,6 bis 0,2 Gew.-% Amitraz.

Das erfindungsgemäße Verfahren eignet sich bevorzugt zur Anwendung gegen alle Entwicklungsstadien der Schadorganismen (Ei, alle Larvenstadien, wie beispielsweise L1, L2, L3, L4, L5 bis Adult), insbesondere bei der Bekämpfung von Homopteren, Dipteren, Lepidopteren und Coleopteren.

Unter dem Begriff 'Bt-Gemüse' sind im Sinne der Erfindung genetisch modifizierte Gemüsepflanzen bzw. -kulturen zu verstehen, in denen ein oder mehrere Gene aus Bacillus thuringiensis enthalten sind, die wiederum insektizid wirksame Proteine codieren und exprimieren. Diese Pflanzen ('Bt-Gemüse') können sowohl im Freiland sowie unter den Bedingungen des Unterglasanbaus kultiviert werden. Zu den entsprechenden Gemüsepflanzen bzw. -kulturen zählen beispielsweise:
Kartoffeln, vorzugsweise
Stärkekartoffeln, Süßkartoffeln und Speisekartoffeln;
Wurzelgemüse, vorzugsweise
Möhren (Karotten), Kohlrüben (Speiserüben, Stoppelrüben, Mairüben, Herbstrüben, Teltower Rübchen), Schwarzwurzeln, Topinambur, Wurzelpetersilie, Patinake, Rettich und Meerrettich;
Knollengemüse, vorzugsweise
Kohlrabi, Rote Bete (Rote Rübe), Sellerie (Knollensellerie), Radies und Radieschen;
Zwiebelgemüse, vorzugsweise
Porree, Lauch und Zwiebeln (Steck- und Samenzwiebel);
Kohlgemüse, vorzugsweise
Kopfkohl (Weißkohl, Rotkohl, Blattkohl, Wirsingkohl), Blumenkohl, Sprossenkohl, Brokkoli, Grünkohl, Marktstammkohl, Meerkohl und Rosenkohl;
Fruchtgemüse, vorzugsweise
Tomaten (Freiland-, Strauch-, Fleisch-, Gewächshaus-, Cocktail-, Industrie- und Frischmarkt-Tomaten), Melonen, Eierfrucht, Auberginen, Paprika (Gemüse- und Gewürzpaprika, Spanischer Pfeffer), Peperoni, Kürbis, Zucchini und Gurken (Freiland-, Gewächshaus-, Schlangen- und Einlegegurken);
Gemüsehülsenfrüchte, vorzugsweise
Buschbohnen (als Schwertbohnen, Perlbohnen, Flageoletbohnen, Wachsbohnen, Trockenkochbohnen mit grün- und gelbhülsigen Sorten), Stangenbohnen (als Schwertbohnen, Perlbohnen, Flageoletbohnen, Wachsbohnen mit grün-, blau- und gelbhülsigen Sorten), Dicke Bohnen (Ackerbohnen, Puffbohnen, Sorten mit weiß und schwarz gefleckten Blüten), Erbsen (Platterbsen, Kichererbsen, Markerbsen, Schalerbsen, Zuckererbsen, Palerbsen, Sorten mit hell- und dunkelgrünem Frischkorn) und Linsen;
Blatt- und Stielgemüse, vorzugsweise
Chinakohl, Kopfsalat, Pflücksalat, Feldsalat, Eisbergsalat, Romanasalat, Eichblattsalat, Endivien, Radicchio, Lollo rosso-Salat, Ruccola-Salat, Chicoree, Spinat, Mangold (Blatt- und Stielmangold) und Petersilie;
Sonstige Gemüse, vorzugsweise
Spargel, Rhabarber, Schnittlauch, Artischocken, Minzen, Sonnenblumen, Knollenfenchel, Dill, Gartenkresse, Senf, Mohn, Erdnuss, Sesam und Salatzichorien.

Alle Pflanzen, die zusammengefasst sind unter dem Begriff 'Bt-Gemüse', sind im Sinne der Erfindung genetisch dahingehend modifiziert, dass sie ein oder mehrere Gene aus Bacillus thuringiensis, welche für ein insektizid wirksames Protein codieren, enthalten und exprimieren.

Bevorzugt sind Bacillus thuringiensis - Kristallproteine aus der Cry-Familie (siehe z. B. Crickmore et al., 1998, Microbiol. Mol. Biol. Rev. 62 : 807-812), die wirksam sind gegen Lepidopteren, Coleopteren und Dipteren.

Besonders bevorzugt sind Gene codierend für die Proteine cry1Aa1, cry1Aa2, cry1Aa3, cry1Aa4, cry1Aa5, cry1Aa6, cry1Aa7, cry1Aa8, cry1Aa9, cry1Aa10, cry1Aa11 cry1 Ab 1, cry1Ab2, cry1Ab3, cry1Ab4, cry01 Ab5, cry1Ab6, cry1Ab7, cry1 Ab8, cry1Ab9, cry1Ab11, cry1Ab12, cry1Ab13, cry1Ab14, cry1Ac1, cry1Ac2, cry1Ac3, cry1Ac4, cry1Ac5, cry1Ac6, cry1Ac7, cry1Ac8, cry1Ac9, cry1Ac10, cry1Ac11, cry1Ac12, cry1Ac13, cry1Ad1, cry1Ad2, cry1Ae1, cry1Af1, cry1Ag1, cry1Ba1, cry1Ba2, cry1Bb1, cry1 Bc1, cry1 Bd 1, cry1 Be1, cry1 Ca 1, cry1 Ca2, cry1 Ca3, cry1 Ca4, cry1 Ca5, cry1 Ca6, cry1 Ca7, cry1 Cb1, cry1 Cb2, cry1 Da1, cry1 Da2, cry1 Db1, cry1 Ea1, cry1 Ea2, cry1 Ea3, cry1 Ea4, cry1 Ea5, cry1 Ea6, cry1 Eb1, cry1 Fa1, cry1 Fa2, cry1 Fb1, cry1 Fb2, cry1 Fb3, cry1Fb4, cry1Ga1, cry1Ga2, cry1Gb1, cry1Gb2, cry1Ha1, cry1Hb1, cry1 la1, cry1Ia2, cry1Ia3, cry1Ia4, cry1Ia5, cry1Ia6, cry1Ib1, cry1Ic1, cry1Id1, cry1Ie1, cry1l-like, cry1Ja1, cry1Jb1, cry1Jc1, cry1Ka1, cry1-like, cry2Aa1, cry2Aa2, cry2Aa3, cry2Aa4, cry2Aa5, cry2Aa6, cry2Aa7, cry2Aa8, cry2Aa9, cry2Ab1, cry2Ab2, cry2Ab3, cry2Ac1, cry2Ac2, cry2Ad1, cry3Aa1, cry3Aa2, cry3Aa3, cry3Aa4, cry3Aa5, cry3Aa6, cry3Aa7, cry3Ba1, cry3Ba2, cry3Bb1, cry3Bb2, cry3Bb3, cry3Ca1, cry4Aa1, cry4Aa2, cry4Ba1, cry4Ba2, cry4Ba3, cry4Ba4, cry5Aa1, cry5Ab1, cry5Ac1, cry5Ba1, cry6Aa1, cry6Ba1, cry7Aa1, cry7Ab1, cry7Ab2, cry8Aa1, cry8Ba1, cry8Ca1, cry9Aa1, cry9Aa2, cry9Ba1, cry9Ca1, cry9Da1, cry9Da2, cry9Ea1, cry9-like, cry10Aa1, cry10Aa2, cry11Aa1, cry11Aa2, cry11Ba1, cry11Bb1, cry12Aa1, cry13Aa1, cry14Aa1, cry15Aa1, cry16Aa1, cry17Aa1, cry18Aa1, cry18Ba1, cry18Ca1, cry19Aa1, cry19Ba1, cry20Aa1, cry21Aa1, cry21Aa2, cry22Aa1, cry23Aa1, cry24Aa1, cry25Aa1, cry26Aa1, cry27Aa1, cry28Aa1, cry28Aa2, cry29Aa1, cry30Aa1, cry31Aa1, cyt1Aa1, cyt1Aa2, cyt1Aa3, cyt1Aa4, cyt1Ab1, cyt1Ba1, cyt2Aa1, cyt2Ba1, cyt2Ba2, cyt2Ba3, cyt2Ba4, cyt2Ba5, cyt2Ba6, cyt2Ba7, cyt2Ba8, cyt2Bb1.

Besonders bevorzugt sind die Subfamilien cry1, cry2, cry3, cry5 und cry9.

Ganz besonders bevorzugt sind cry1Ab, cry1Ac, cry3A, cry3B und cry9C.

Weiterhin bevorzugt ist es, Pflanzen einzusetzen, die Gene für mehrere Bt-Proteine enthalten.

Die transgenen Kulturpflanzen können neben der Expression von Toxinen aus Bacillus thuringiensis (Bt) zur Isektenresistenz auch weitere transgene Eigenschaften aufweisen, z. B. weitere Insektenresistenzen (z. B. durch Expression eines Protease- bzw. Peptidase-Inhibitors, vgl. WO-A 95/35031), Herbizidresistenzen (z. B. gegen Glufosinate oder Glyphosate durch Expression des pat- oder bar-Gens) oder auch Nematoden-, Pilz- oder Virusresistenzen (z. B. durch Expression einer Glucanase, Chitinase) oder auch in ihren metabolischen Eigenschaften genetisch modifiziert sein, so dass eine qualitative und/oder quantitative Änderung von Inhaltsstoffen (z. B. durch Modifikationen des Energie-, Kohlenhydrat-, Fettsäure- oder Stickstoffwechsels oder diese beeinflussende Metabolitflüsse) resultiert. Bevorzugt sind beispielsweise Bt-Gemüsepflanzen, die zusätzlich eine Glufosinate- oder Glyphosate-Resistenz aufweisen.

Bt-Gemüsepflanzen sind einschließlich Methoden zu ihrer Herstellung, ausführlich beschrieben in beispielsweise Barton et al., 1987, Plant Physiol. 85 : 1103-1109; Vaeck et al., 1987, Nature 328 : 33-37; Fischhoff et al., 1987, Bio/Technology 5 : 807-813.

Bt-Gemüsepflanzen sind weiterhin in verschiedenen Variationen kommerziell erhältlich, beispielsweise die Kartoffelsorte NewLeaf® von der Firma Monsanto. Weitere Gemüsesorten sind in der Entwicklung bzw. Prüfung, wie beispielsweise eine Tomatensorte mit Resistenz gegen Lepidopteren von der Firma Monsanto.

Wege zur Herstellung transgener Pflanzen, die im Vergleich zu natürlich vorkommenden Pflanzen modifizierte Eigenschaften aufweisen, bestehen beispielsweise in der Anwendung der im folgenden genannten Verfahren: Willmitzer, 1993, Transgenic plants, In: Biotechnology, A Multivolume Comprehensive Treatise, Rehm et al. (eds.), Vol.2, 627-659, VCH Weinheim, Germany; McCormick et al., 1986, Plant Cell Reports 5 : 81-84; EP-A 0221044; EP-A 0131624.

Zahlreiche molekularbiologische Techniken, mit denen neue transgene Pflanzen mit veränderten Eigenschaften hergestellt warden können, sind dem Fachmann bekannt; siehe z. B. Sambrook et al., 1989, "Molecular Cloning: A Laboratory Manual", 3rd Ed., Cold Spring Harbor Laboratory Press, Cold Spring Harbor, NY; Winnacker, 1996, "Gene und Klone", 2. Aufl., VCH Weinheim oder Christou, 1996, Trends in Plant Science 1 : 423-431.

Für derartige gentechnische Manipulationen können geeignete Nucleinsäuremoleküle z. B. mittels geeigneter Vektoren, die eine Mutagenese oder eine Sequenzveränderung durch Rekombination von DANN-Sequenzen erlauben, in Pflanzen bzw. pflanzliche Zellen eingebracht werden. Mit Hilfe der obengenannten Standardverfahren können z. B. Basenaustausche vorgenommen, Teilsequenzen entfernt oder natürliche oder synthetische Sequenzen hinzugefügt werden. Es können auch z. B. die natürlich vorkommenden Gene vollständig durch heterologe bzw. synthetische Gene vorzugsweise unter der Kontrolle eines in Pflanzenzellen aktiven Promoters ersetzt werden (,gene replacement'). Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codierenden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment oder zu einem bestimmten Zeitpunkt (zu einem bestimmten Stadium oder chemisch oder biologisch induziert) gewährleisten (z. B. Transit- oder Signalpeptide, zeit- oder ortspezifische Promotoren). Derartige Sequenzen sind dem Fachmann bekannt (siehe beispielsweise Braun et al., 1992, EMBO J. 11 : 3219-3227; Wolter et al., 1988, Proc. Natl. Acad. Sci. USA 85 : 846-850; Sonnewald et al., 1991, Plant J. 1 : 95-106).

Die transgenen Pflanzenzellen können nach bekannten Techniken zu ganzen Pflanzen regeneriert werden.

Auf diese Weise sind transgene Pflanzen erhältlich, die veränderte Eigenschaften durch Überexpression, Suppression oder Inhibierung homologer (d. h. endogener) Gene oder Gensequenzen oder Expression heterologer (d. h. exogener) Gene oder Gensequenzen aufweisen.

Das erfindungsgemäße Verfahren eignet sich zur Bekämpfung einer Vielzahl von Schadorganismen, die insbesondere in Gemüse auftreten, insbesondere Insekten und Spinnentieren, ganz besonders bevorzugt Insekten. Zu den erwähnten Schädlingen gehören:
Aus der Ordnung der Isopoda zum Beispiel Armadillidium spp., Oniscus spp., Porcellio spp..
Aus der Ordnung der Diplopoda zum Beispiel Blaniulus spp..
Aus der Ordnung der Chilopoda zum Beispiel Geophilus spp., Scutigera spp..
Aus der Ordnung der Symphyla zum Beispiel Scutigerella spp..
Aus der Ordnung der Thysanura zum Beipiel Lepisma spp..
Aus der Ordnung der Collembola zum Beispiel Onychiurus spp..
Aus der Ordnung der Orthoptera zum Beispiel Blattella spp., Periplaneta spp., Leucophaea spp., Acheta spp., Gryllotalpa spp., Locusta migratoria migratorioides, Melanoplus spp., Schistocerca spp..
Aus der Ordnung der Dermaptera zum Beispiel Forficula spp..
Aus der Ordnung des Isoptera zum Beispiel Reticulitermes spp., Reticulitermes spp., Coptotermes spp..
Aus der Ordnung der Thysanoptera zum Beispiel Frankliniella spp., Kakothrips spp., Hercinothrips spp., Scirtothrips spp., Taeniothrips spp., Thrips spp..
Aus der Ordnung der Heteroptera zum Beispiel Eurygaster spp., Stephanitis spp., Lygus spp., Aelia spp., Eurydema spp., Dysdercus spp., Piesma spp..
Aus der Ordnung der Homoptera zum Beispiel Aleurodes spp., Bemisia spp., Trialeurodes spp., Brevicoryne spp., Cryptomyzus spp., Aphis spp., Eriosoma spp., Hyalopterus spp., Phylloxera spp., Pemphigus spp., Macrosiphum spp., Myzus spp., Phorodon spp., Rhopalosiphum spp., Empoasca spp., Euscelis spp., Eulecanium spp., Saissetia spp., Aonidiella spp., Aspidiotus spp., Nephotettix spp., Laodelphax spp., Nilaparvata spp., Sogatella spp., Pseudococcus spp., Psylla spp., Aphrophora spp., Aeneolamia spp..
Aus der Ordnung der Lepidoptera zum Beispiel Pectinophora spp., Bupalus spp., Cheimatobia spp., Cnephasia spp., Hydraecia spp., Lithocolletis spp., Hyponomeuta spp., Plutella spp., Plutella xylostella, Malacosoma spp., Euproctis spp., Lymantria spp., Bucculatrix spp., Phytometra spp., Scrobipalpa spp., Phthorimaea spp., Gnorimoschema spp., Autographa spp., Evergestis spp., Lacanobia spp., Cydia spp., Pseudociaphila spp., Phyllocnistis spp., Agrotis spp., Euxoa spp., Feltia spp., Earias spp., Heliothis spp., Helicoverpa spp., Bombyx spp., Laphygma spp., Mamestra spp., Panolis spp., Prodenia spp., Spodoptera spp., Trichoplusia spp., Carpocapsa spp., Pieris spp., Chilo spp., Ostrinia spp., Pyrausta spp., Ephestia spp., Galleria spp., Cacoecia spp., Capua spp., Choristoneura spp., Clysia spp., Hofmannophila spp., Homona spp., Tineola spp., Tinea spp., Tortrix spp..
Aus der Ordnung der Coleoptera zum Beispiel Anobium spp., Rhizopertha spp., Bruchidius spp., Acanthoscelides spp., Hylotrupes spp., Aclypea spp., Agelastica spp., Leptinotarsa spp., Psylliodes spp., Chaetocnema spp., Cassida spp., Bothynoderes spp., Clivina spp., Ceutorhynchus spp., Phyllotreta spp., Apion spp., Sitona spp., Bruchus spp., Phaedon spp., Diabrotica spp., Psylloides spp., Epilachna spp., Atomaria spp., Oryzaephilus spp., Anthonomus spp., Sitophilus spp., Otiorhynchus spp., Cosmopolites spp., Ceuthorrynchus spp., Hypera spp., Dermestes spp., Trogoderma spp., Anthrenus spp., Attagenus spp., Lyctus spp., Meligethes spp., Ptinus spp., Niptus spp., Gibbium spp., Tribolium spp., Tenebrio spp., Agriotes spp., Conoderus spp., Melolontha spp., Amphimallon spp., Costelytra spp..
Aus der Ordnung der Hymenoptera zum Beispiel Diprion spp., Hoplocampa spp., Lasius spp., Monomorium spp., Vespa spp..
Aus der Ordnung der Diptera zum Beispiel Drosophila spp., Chrysomyxa spp., Hypoderma spp., Tannia spp., Bibio spp., Oscinella spp., Phorbia spp., Pegomyia spp., Ceratitis spp., Dacus spp., Tipula spp..
Aus der Klasse der Arachnida zum Beispiel Scorpio maurus, Latrodectus mactans. Aus der Ordnung der Acarina zum Beispiel Acarus spp., Bryobia spp., Panonychus spp., Tetranychus spp., Eotetranychus spp., Oligonychus spp., Eutetranychus spp., Eriophyes spp., Phyllocoptruta spp., Tarsonemus spp., Rhipicephalus spp., Rhipicephalus spp., Ixodes spp., Amblyomma spp..
Aus der Klasse der Helminthen zum Beispiel Meloidogyne spp., Heterodera spp., Globodera spp., Radopholus spp., Pratylenchus spp., Tylenchulus spp., Tylenchorhynchus spp., Rotylenchus spp., Heliocotylenchus spp., Belonoaimus spp., Longidorus spp., Trichodorus spp., Xiphinema spp., Ditylenchus spp., Aphelenchoides spp., Anguina spp..
Aus der Klasse der Gastropoda zum Beispiel Deroceras spp., Arion spp., Lymnaea spp., Galba spp., Succinea spp..

Bevorzugt eignet sich das erfindungsgemäße Verfahren zur Bekämpfung von Agriotes spp., Melolontha spp., Aphis spp., Cnephasia spp., Ostrinia spp., Agrotis spp., Hydraecia spp., Tipula spp., Myzus spp., Bemisia spp., Trialeurodes spp., Oscinella spp., Tetranychus spp., Lygus spp., Leptinotarsa spp., Psylliodes spp., Phytometra spp., Deroceras spp., Psylla spp., Blaniulus spp., Onychiurus spp., Piesma spp., Atomaria spp., Aclypea spp., Chaetocnema spp., Cassida spp., Bothynoderes spp., Clivina spp., Scrobipalpa spp., Phthorimaea spp., Gnorimoschema spp., Mamestra spp., Autographa spp., Arion spp., Gryllotalpa spp., Eurydema spp., Meligethes spp., Ceutorhynchus spp., Phyllotreta spp., Plutella xylostella, Evergestis spp., Lacanobia spp., Pieris spp., Forficula spp., Hypera spp., Apion spp., Otiorhynchus spp., Sitona spp., Acanthoscelides spp., Kakothrips spp., Bruchus spp., Cydia spp., Thrips spp., Pseudociaphila spp., Heliothis spp., Helicoverpa spp., Prodenia spp., Spodoptera spp., Chilo spp. und Diabrotica spp..

Die Erfindung wird durch die folgenden Beispiele näher erläutert, ohne sie dadurch einzuschränken.

### Beispiel

### Plutella xylostella (Gemüse-Motte, Kohl-Schabe)

Sieben Wochen alte Blumenkohl-Pflanzen (gewöhnlicher Blumenkohl, Sorte Marner Allfrüh) und Bt-Blumenkohl (Bt resistenter Stamm, enthält cry9C) wurden mit Hilfe eines Tracksprayers (200 I/ha) mit den zu testenden Insektiziden besprüht. Nach dem Trocknen wurden Pflanzen mit jeweils 5 L2-Larven und 5 L3-Larven von Plutella xylostella infiziert. Nach 2, 4 und 7 Tagen (DAA) wurden Fraßschaden und Mortalität bonitiert. Die Prüfung wurde im Gewächshaus bei 23°C und 60% rel. Feuchte durchgeführt.

| | | % Fraßschäden / % Mortalität | | | | | | | | | | | |
|---|---|---|---|---|---|---|---|---|---|---|---|---|---|
| Verbindung | | 2 DAA | | | | 4 DAA | | | | 7 DAA | | | |
| g Wirkstoff/ha | | Blumenkohl | | Bt.-Blumenkohl | | Blumenkohl | | Bt.-Blumenkohl | | Blumenkohl | | Bt.-Blumenkohl | |
| Kontrolle | -- | 10 | 20 | 10 | 0 | 15 | 20 | 15 | 20 | 15 | 20 | 15 | 30 |
| | | | | | | | | | | | | | |
| Tolfenpyrad | 100 | 5 | 0 | 10 | 20 | 5 | 20 | 10 | 100 | 5 | 0 | 10 | 100 |
| EC15 | 30 | 10 | 0 | 0 | 60 | 10 | 0 | 0 | 100 | 10 | 0 | 0 | 100 |
| | 10 | 10 | 0 | 10 | 0 | 10 | 0 | 10 | 50 | 10 | 0 | 10 | 50 |
| | 3 | 10 | 0 | 3 | 10 | 10 | 0 | 10 | 30 | 10 | 0 | 10 | 50 |
| | | | | | | | | | | | | | |
| Fipronil | 100 | 1 | 100 | 0 | 100 | 1 | 100 | 0 | 100 | 1 | 100 | 0 | 100 |
| SC05 | 30 | 4 | 70 | 0 | 100 | 5 | 100 | 0 | 100 | 5 | 100 | 0 | 100 |
| | 10 | 8 | 20 | 0 | 100 | 8 | 20 | 0 | 100 | 10 | 50 | 0 | 100 |
| | 3 | 10 | 20 | 0 | 100 | 10 | 20 | 0 | 100 | 12 | 20 | 0 | 100 |
| | 1 | 10 | 0 | 0 | 70 | 10 | 0 | 0 | 100 | 12 | 0 | 0 | 100 |
| | | | | | | | | | | | | | |
| Thiodicarb | 100 | 10 | 0 | 0 | 100 | 10 | 0 | 0 | 100 | 10 | 0 | 0 | 100 |
| SC33 | 30 | 10 | 0 | 0 | 100 | 10 | 0 | 0 | 100 | 15 | 0 | 0 | 100 |
| | 10 | 10 | 0 | 0 | 90 | 10 | 0 | 0 | 100 | 15 | 0 | 0 | 100 |
| | 3 | 10 | 0 | 0 | 100 | 10 | 0 | 0 | 100 | 15 | 0 | 0 | 100 |
| | 1 | 10 | 0 | 0 | 100 | 10 | 0 | 0 | 100 | 15 | 0 | 0 | 100 |
| | | | | | | | | | | | | | |
| Acetamiprid | 30 | 10 | 0 | 0 | 100 | 12 | 0 | 0 | 100 | 15 | 0 | 0 | 100 |
| WP20 | 10 | 10 | 0 | 0 | 100 | 12 | 0 | 0 | 100 | 15 | 0 | 0 | 100 |
| | | | | | | | | | | | | | |

Bei allen vier Wirkstoffen ist ein signifikanter Effekt deutlich erkennbar.

## Patentansprüche

1. Verfahren zur Bekämpfung von Schadorganismen in gentechnisch veränderten Gemüsepflanzen, die ein aus Bacillus thuringiensis abgeleitetes Gen enthalten, welches für ein insektizid wirksames Protein codiert und dieses exprimiert, **dadurch gekennzeichnet, dass** man eine insektizid wirksame Menge an einer oder mehreren Verbindungen aus den folgenden Gruppen (a) bis (f) auf die Pflanzen, deren Saat- oder Vermehrungsgut und/oder deren Anbaufläche aufbringt:
a) insektizide Organophosphorverbindungen aus der Gruppe:
Acephate, Azinphos-ethyl, Azinphos-methyl, Cadusafos, Chlorfenvinphos, Chlormephos, Chlorpyrifos, Demeton-S-methyl, Diazinon, Dicrotophos, Dimethoate, Disulfoton, Ethion, Ethoprophos, Etrimfos, Fonofos, Isazofos, Isofenphos, Malathion, Methamidophos, Methidathion, Mevinphos, Monocrotophos, Omethoate, Parathion, Phenthoate, Phorate, Phosalone, Phosmet, Phosphamidon, Phoxim, Pirimiphosmethyl, Prothiofos, Pyridaphenthion, Quinalphos, Terbufos, Tetrachlorvinphos und Triazophos;
c) insektizide Carbamate aus der Gruppe:
Alanycarb, Aldicarb, Amitraz, Bendiocarb, Benfuracarb, Butocarboxim, Carbaryl, Carbofuran, Carbosulfan, Ethiofencarb, Formetanate, Furathiocarb, Isoprocarb, Methiocarb, Methomyl, Oxamyl, Pirimicarb, Propoxur, Thiofanox, Thiodicarb und Trimethacarb;
d) Biopestizide aus der Gruppe:
Bacillus thuringiensis, Bacillus firmus, Granulose und Kernpolyeder-Viren, Beauveria bassiana, Beauveria brogniartii und Baculoviren, wie Autographa california;
e) insektizide Wachstumsregulatoren aus der Gruppe:
Chlorfluazuron, DBI-3204, Diflubenzuron, Flucycloxuron, Flufenoxuron, Hexaflumuron, , Novaluron, Methoxyfenozide, Teflubenzuron, Tebufenozide und Triflumuron;
f) Sonstige:
Abamectin, Bensultap, Cartap, Chlordane, Chlorfenapyr, DNOC, Endosulfan, Fipronil,
Ethiprole, , , Phosphine, Oleinsäure/Fettsäuren, , Thiocyclam, IKI-220, Tolfenpyrad, ,
Spinosad, Silafluofen und.

2. Verfahren nach Anspruch 1, **dadurch gekennzeichnet, dass** man eine Verbindung aus der Gruppe insektizide Organophosphorverbindungen (a), , insektizide Carbamate (c), insektizide Wachstumsregulatoren (e), Abamectin, Acetamiprid, Chlorfenapyr, Endosulfan, Fipronil, Ethiprole, , , Spinosad, Silafluofen, , , Tolfenpyrad, IKI-220 und Bacillus thuringiensis verwendet.

3. Verfahren nach Anspruch 2, **dadurch gekennzeichnet, dass** man eine Verbindung aus der Gruppe Ethoprophos, Malathion, Parathion, Phosalone, Pirimiphos-methyl, Triazophos, Acrinathrin, Deltamethrin, Tefluthrin, Aldicarb, Amitraz, Bendiocarb, Carbaryl, Carbofuran, Oxamyl, Pirimicarb, Thiodicarb, Diflubenzuron, , Novaluron, Methoxyfenozide, Tebufenozide, Abamectin, Acetamiprid, Chlorfenapyr, Endosulfan, Fipronil, Ethioprole... Spinosad, Silafluofen... Tolfenpyrad, IKI-220 und Bacillus thuringiensis verwendet.

4. Verfahren nach einem oder mehreren der Ansprüche 1 bis 3, **dadurch gekennzeichnet, dass** man eine Mischung aus zwei oder mehr der insektizid wirksamen Verbindungen einsetzt.

5. Verfahren nach einem oder mehreren der Ansprüche 1 bis 4, **dadurch gekennzeichnet, dass** man die insektizid wirksame Verbindung in einer Aufwandmenge von 0,0001 bis 5,0 kg/ha aufbringt.

6. Verfahren nach einem oder mehreren der Ansprüche 1 bis 5, **dadurch gekennzeichnet, dass** man insektizid wirksame Verbindung als eine 0,00001 bis 95 gew.-%ige Formulierung einsetzt.

7. Verfahren nach einem oder mehreren der Ansprüche 1 bis 6, **dadurch gekennzeichnet, dass** das insektizid wirksame Protein in der Gemüsepflanze ein Kristallprotein aus der Subfamilie cry1, cry2, cry3, cry5 und/oder cry9 ist.

8. Verfahren nach Anspruch 7, **dadurch gekennzeichnet, dass** das insektizid wirksame Protein in der Gemüsepflanze cry1Ab, cry1Ac, cry3A, cry3B und/oder cry9C ist.

9. Verfahren nach einem oder mehreren der Ansprüche 1 bis 8, **dadurch gekennzeichnet, dass** man Gemüsepflanzen verwendet, die eine Glufosinate- oder Glyphosat-Resistenz aufweisen.

10. Verfahren nach einem oder mehreren der Ansprüche 1 bis 9, **dadurch gekennzeichnet, dass** die Schadorganismen Insekten sind, die zu den Ordnungen Homoptera, Lepidoptera und/oder Coleoptera gehören.

11. Verfahren nach einem oder mehreren der Ansprüche 1 bis 10, **dadurch gekennzeichnet, dass** man die insektizid wirksame Verbindung gegen Eier und/oder gegen Larven im L1 und/oder L2 und/oder L3 und/oder L4 Stadium und/oder L5 Stadium und/oder gegen adulte Tiere der Schadorganismen einsetzt.

12. Verfahren nach einem oder mehreren der Ansprüche 1 bis 11, **dadurch gekennzeichnet, dass** man neben einer oder mehreren insektizid wirksamen Verbindungen aus der Gruppe (a) bis (f), eine oder mehrere weitere insektizid, fungizid oder herbizid wirksame Verbindungen einsetzt.

13. Verwendung von Verbindungen aus den folgenden Gruppen (a) bis (f)
a) insektizide Organophosphorverbindungen aus der Gruppe:
Acephate, Azinphos-ethyl, Azinphos-methyl, Cadusafos, Chlorfenvinphos, Chlormephos, Chlorpyrifos, Demeton-S-methyl, Diazinon, Dicrotophos, Dimethoate, Disulfoton, Ethion, Ethoprophos, Etrimfos, Fonofos, Isazofos, Isofenphos, Malathion, Methamidophos, Methidathion, Mevinphos, Monocrotophos, Omethoate, Parathion, Phenthoate, Phorate, Phosalone, Phosmet, Phosphamidon, Phoxim, Pirimiphos-methyl, , Prothiofos, Pyridaphenthion, Quinalphos, Terbufos, Tetrachlorvinphos und Triazophos;
c) insektizide Carbamate aus der Gruppe:
Alanycarb, Aldicarb, Amitraz, Bendiocarb, Benfuracarb, Butocarboxim, Carbaryl, Carbofuran, Carbosulfan, Ethiofencarb, Formetanate, Furathiocarb, Isoprocarb, Methiocarb, Methomyl, Oxamyl, Pirimicarb, Propoxur, Thiofanox, Thiodicarb und Trimethacarb;
d) Biopestizide aus der Gruppe:
Bacillus thuringiensis, Bacillus firmus, Granulose und Kernpolyeder-Viren, Beauveria bassiana, Beauveria brogniartii und Baculoviren, wie Autographa california;
e) insektizide Wachstumsregulatoren aus der Gruppe:
Chlorfluazuron, DBI-3204, Diflubenzuron, Flucycloxuron, Flufenoxuron, Hexaflumuron, , Novaluron, Methoxyfenozide, Teflubenzuron, Tebufenozide und Triflumuron;
f) Sonstige:
Abamectin, Besultap, Cartap, Chlordane, Chlorfenapyr, DNOC, Endosulfan, Fipronil, Ethiprole, , Phosphine, Oleinsäure/Fettsäuren, , Thiocyclam, IKI-220, Tolfenpyrad, , Spinosad, Silafluofen und;
zur Bekämpfung von Schadorganismen in gentechnisch veränderten Gemüsepflanzen, die ein aus Bacillus thuringiensis abgeleitetes Gen enthalten, welches ein insektizid wirksames Protein codiert und dieses exprimiert.
